# EUROPEAN PATENT SPECIFICATION

(11) **EP 2 092 057 B1**
(45) Date of publication and mention of the grant of the patent: **15.10.2014**
(21) Application number: 07840085.0
(22) Date of filing: 20.11.2007
(51) Int. Cl.: C12N 5/071, A61K 35/12

(54) **USE OF BONE MARROW CELLS FOR LONG TERM CULTURE OF PANCREATIC ISLET CELLS**
VERWENDUNG VON KNOCHENMARKZELLEN FÜR DIE LANGZEITKULTUR PANKREATISCHER INSELZELLEN
UTILISATION DES CELLULES DE LA MOELLE OSSEUSE POUR UNE CULTURE A LONG TERME DES CELLULES DES ILOTS PANCREATIQUES

(30) Priority: 21.11.2006 US 860637 P
(43) Date of publication of application: 26.08.2009
(73) Proprietor: Roger Williams Hospital, Providence, RI 02908 (US)
(72) Inventor: LUO, Luguang, Seekonk, MA 02771 (US)
(74) Representative: Reitstötter Kinzebach
(86) International application number: PCT/US2007/024258
(87) International publication number: WO 2008/063640

(56) References cited:
- EP-A1- 1 498 478
- LIU ET AL: "Adult Bone Marrow Cells Transdifferentiate into Endocrine Cells During Pancreatic Regeneration", BLOOD,, vol. 102, no. 11, 1 November 2003 (2003-11-01), page 338A, XP008108956,
- HESS D ET AL: "BONE MARROW-DERIVED STEM CELLS INITIATE PANCREATIC REGENERATION", NATURE BIOTECHNOLOGY, NATURE PUBLISHING GROUP, NEW YORK, NY, US, vol. 21, no. 7, 1 July 2003 (2003-07-01), pages 763-770, XP001156912, ISSN: 1087-0156, DOI: DOI:10.1038/NBT841
- LUO ET AL: "Allogeneic bone marrow supports human islet beta cell survival and function over six months", BIOCHEMICAL AND BIOPHYSICAL RESEARCH COMMUNICATIONS, ACADEMIC PRESS INC. ORLANDO, FL, US, vol. 361, no. 4, 10 September 2007 (2007-09-10), pages 859-864, XP022240269, ISSN: 0006-291X, DOI: DOI:10.1016/J.BBRC.2007.07.105
- SORDI V ET AL: "Bone marrow mesenchymal stem cells express restricted set of functi a restricted set of functionally active chemokine receptors capable of promoting migration to pancreatic islets", BLOOD, AMERICAN SOCIETY OF HEMATOLOGY, US, vol. 106, no. 2, 15 July 2005 (2005-07-15) , pages 419-427, XP003019019, ISSN: 0006-4971, DOI: DOI:10.1182/BLOOD-2004-09-3507
- LIU ET AL.: 'Adult Bone Marrow Cells Transdifferentiate into Endocrine Cells During Pancreatic Regeneration' BLOOD vol. 102, no. 11, November 2003, page 338A, XP008108956
- LUCAS-CLERC C. ET AL.: 'Long-term Culture of Human Pancreatic Islets in an Extracellular Matrix: Morphological and Metabolic Effects' MOLECULAR AND CELLULAR ENDOCRINOLOGY vol. 94, no. 1, 1993, pages 9 - 20, XP023488574

## Description

The present invention was made with United States government support under National Institutes of Health (NIH) grant number P20RR018757. Accordingly, the United States government has certain rights to the invention.

### FIELD OF THE INVENTION

The invention generally relates to the field of pancreatic islet cell culture. More particularly, the invention relates to the use of bone marrow cells for long term culture and possible expansion of pancreatic islet cells prior to transplant. The invention also includes the use of islet cells grown in the presence of bone marrow cells to replace human islet function *in vivo* in a subject.

### BACKGROUND

Diabetes mellitus represents a major and growing world-wide health problem [1] Standard insulin-based therapeutic approaches, while managing blood sugar levels, are not curative and patients are still subject to many long-term complications of the disease [2, 3]. Pancreatic transplants can cure the disease, but are limited by tissue availability and response to immunosuppressive therapies [4]. In order to maintain the viability of the islet cells, relatively rapid harvesting of the pancreas from the donor is required. Standard transplant immunosuppressive regimens are not always effective with pancreatic transplants. Moreover, immunosuppressive therapies can result in further damage to kidneys, which frequently have compromised function in those requiring pancreatic transplants. Alternatively, transplant of islet β cells can be effective and require a less drastic immunosuppressive regimen; however, islet cells are less available than pancreases [5]. A successful islet cell transplant often requires cells harvested from two or three donors, further limiting the number of individuals who can be treated by this method. The isolation of the islet β cells for transplant is also non-trivial.

Events sustained during the isolation, storage and transport of pancreatic islets are associated with cell death that leads to a loss of islet function and limits the therapeutic potential of islet transplantation for diabetic patients. Critical problems with β cell islet transplants approaches relate to difficulties in maintaining viable islets *in vitro* or *in vivo* and a failure to expand islet tissue in *in vitro* culture. Studies have demonstrated that islet cell transplants are most successful when the cells are not cultured between harvest from the pancreas which is performed as soon as possible after harvest of the pancreas from the donor. Not surprisingly, transplants are also most successful at centers that do the most transplants. Due to this lack of robust culture conditions, donors for islet cell transplantation must be close to a major transplant center at the time of tissue harvest, and recipients must live near major transplant centers, often for extended periods of time, waiting for more than one donor.

Liu et al. ("Adult Bone Marrow Cells Transdifferentiate into Endocrine Cells During Pancreatic Regeneration," Blood 2003; 102(11): 338A; "D1/Liu") describes a study of pancreatic regeneration in which adult bone marrow cells can transdifferentiate into ductal and endocrine cells. Liu used bone marrow mesenchymal cells as a feeder mono-layer to co-culture with islets to test insulin responses. Hess et al. ("Bone marrow-derived stem cells initiate pancreatic regeneration." Nat. Biotech 2003; 21(7): 763-770; "D2/Hess") describes in vivo experiments involving engraftment of bone marrow cells to pancreatic tissue to treat diabetes.

### SUMMARY OF THE INVENTION

The invention relates to the methods defined in claim 1 to 10..

Thus, the invention includes methods for maintaining pancreatic islet β cell viability, structure, and/or function in culture for a sustained period. The methods include culturing of pancreatic islet β cells with bone marrow cells. Bone marrow cells were found to promote islet β cell growth and viability, and improve β islet cell function and morphology while reducing inflammatory cytokine release and apoptosis. Islet cells were shown to retain islet β cell function as demonstrated by basal and induced insulin release. Cord blood cells and isolated peripheral CD34+ blood cells were unable to support β islet cell growth or increase survival.

The invention includes methods for the expansion of pancreatic islet β cells in culture by culturing of pancreatic islet β cells with a plurality of bone marrow cells, including a method to decrease apoptosis in co-culture. Bone marrow cells were found to promote islet β cell growth at least partially by reducing apoptosis, but also by increasing islet β cells numbers. The ability to expand islet cells is necessary to reduce the need for multiple donors for a single recipient, potentially allowing for broader use of islet β cell transplants.

The invention includes methods for promoting islet aggregation in culture by culturing of pancreatic islet β cells with a plurality of bone marrow cells. Bone marrow cells stimulate the process of islet aggregation.

The invention further includes a method for decreasing release of cytokines and other inflammatory modulators from islet β cells in culture. The methods include culturing of a pancreatic islet β cells with a bone marrow cell. Co-culture of islet β cells with bone marrow cells reduced release of inflammatory cytokines such as interleukin (IL)-1β, as compared to islet β cells grown without bone marrow cells. This may result in a reduced inflammatory response in patients in response to islet β cell transplant.

The invention includes methods for increasing gene expression of endocrine cell-specific genes including pancreas-specific transcription factors by culturing of pancreatic islet β cells with a plurality of bone marrow cells. The factors including endocrine cell specific genes of GCG (glucagon, α-cell gene), INS (insulin, β-cell gene), and SST (somatostatin, γ-cell gene); and transcription factors for β cell pancreatic and duodenal homeoboxl (PDX1 or IPF1), Neurogenin 3 (NGN3), paired box gene 6 (PAX6), islet-1 (ISL1), v-maf musculoaponeurotic fibrosarcoma oncogene homolog A (MAFa), and Mist 1. The methods include culturing of pancreatic islet β cells with a bone marrow cell. The method further includes increasing expression of NGN3 for increasing β cell regeneration. The method further includes promoting long term survival in β cells by increasing expression of at least one of PDX1, IPF1, NGN3, PAX6, ISL1, and MAFa. The method also includes a method of promoting organization of new pancreatic tissue by increasing expression of Mist1.

Furthermore, the application describes a method for treatment of an individual in need of an islet cell transplant comprising obtaining islet β cells, culturing islet cells in the presence of bone marrow cells, and implanting the combined islet β cells and bone marrow cells into the subject in need of treatment. This method includes transplantation of bone marrow to improve and/or replace pancreatic cell function *in vivo* by direct implantation of islet cells cultured in the presence of bone marrow, preferably autologous bone marrow, to improve diminished or lost pancreatic cell function in the subject in need of treatment. The subject in need of an islet β cell transplant may be an individual suffering from type I or type II diabetes. Diabetes can be a result of damage to the pancreas due to disease, or due to removal of at least a portion of the pancreas as in pancreatic cancer. The method can further include selecting or identifying a subject in need of an islet cell transplant, or improved or replaced islet cell function. The method can also include monitoring the subject for improved pancreatic activity.

Furthermore, the application describes kits to practice the methods of the invention. The kits can include, for example, a bone marrow cell or instructions on how to obtain a bone marrow cell, with instructions on how to culture pancreatic cells by the method of the invention. Kits can further include reagents for culturing islet cells in the presence of bone marrow cells, or reagents to determine the viability, function, and/or characteristics of the islet cells grown in culture with the bone marrow cells.

### BRIEF DESCRIPTION OF THE DRAWINGS

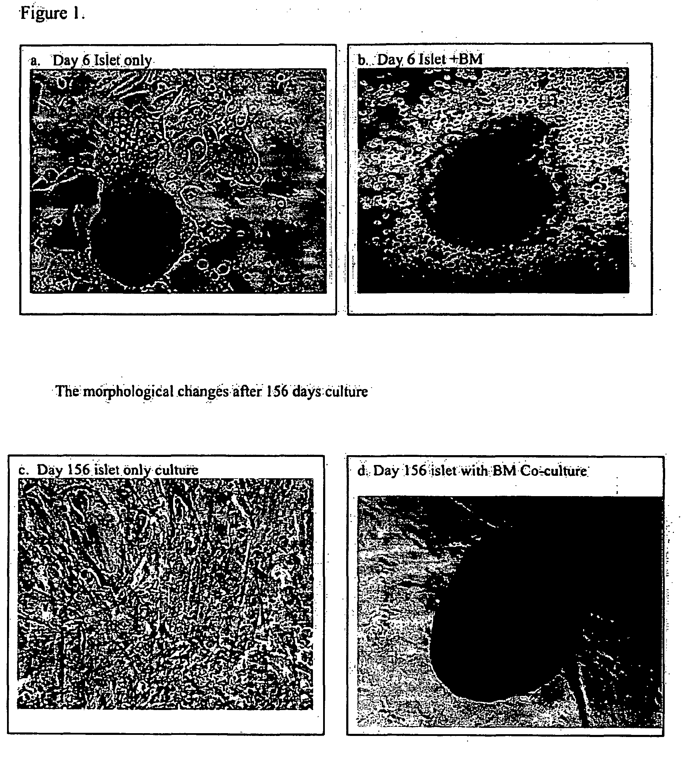

**Figures 1a-1d****.** Phase contrast microscopy of islet cells in culture with or without bone marrow cells for 6 days or 156 days. (Image magnification 20x) Figure 1a shows islet β cells alone in culture for 6 days. Figure 1b shows islet β cells in culture with bone marrow cells for six days. Figure 1c shows islet β cells alone in culture for 156 days. Figure 1d shows islet β cells in culture with bone marrow cells for 156 days.

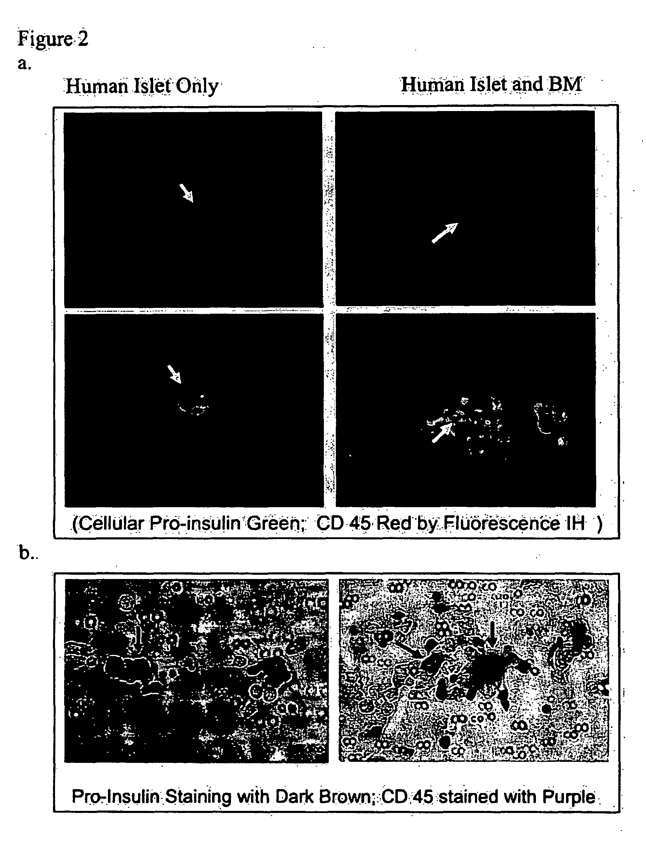

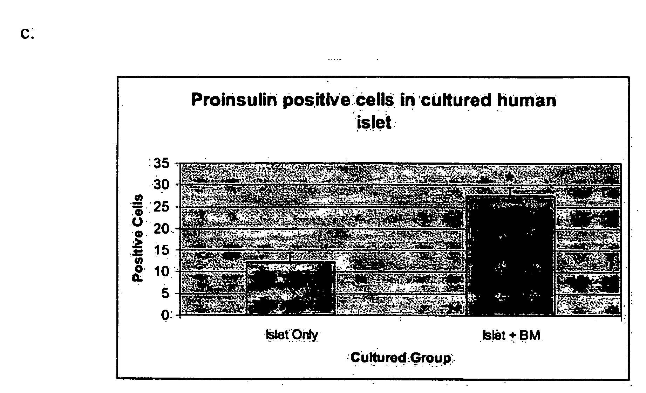

**Figures 2a****-c.** Immunofluorescence and immunohistochemical analyses of islet β cells and bone marrow cells using cell type specific markers. Figure 2a shows fluorescent immunohistochemical staining with antibodies of proinsulin (indicated by arrows), CD45 (bone marrow cells, indicated by fine arrow) (bottom panels) and nuclear staining with DAPI. Figure 2b shows immunohistochemical staining for proinsulin (indicated by thicker arrow) and CD45 (indicated by thinner arrow). (Image magnification x 40). Figure 2c is a bar graph showing the number of proinsulin positive cells in islet alone cultures as compared to bone marrow-islet cell co-cultures at 28 days. (*= p < 0.01, n = 6)

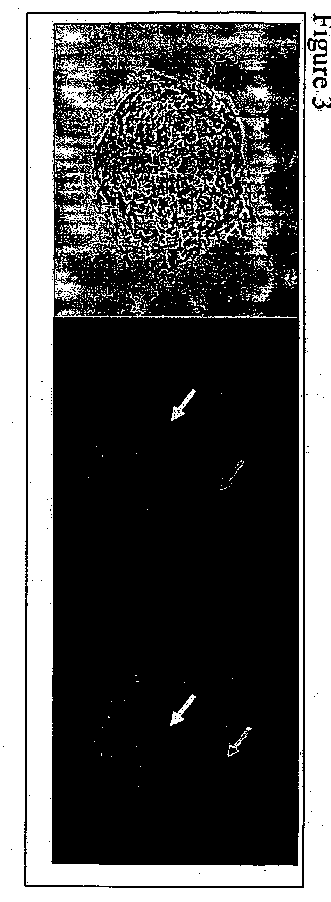

**Figure 3****.** Immunofluorescence analysis of islet α and β cells using cell type specific markers at 153 days in culture. Images are phase contrast microscopy (left panel), and confocal microscopy at the surface (center panel) and middle (right panel) of the islet. Alpha cells stained with antibodies to glucagons (indicated by light arrow), and β cells are stained with antibodies to proinsulin (indicated by dark arrow).

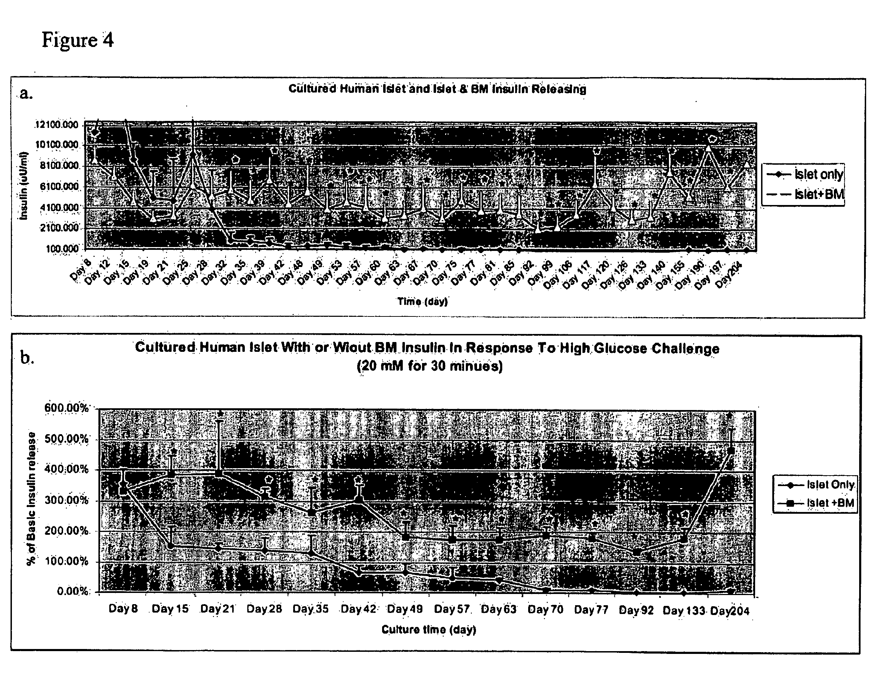

**Figures 4a****-b.** Functional analysis of islet β cells grown with or without bone marrow cells. Figure 4a is a graph of basal insulin secretion over a series of time points. Figure 4b is a graph of insulin release in response to high glucose over a series of time points up to 204 days. (*= p < 0.01, n = 6).

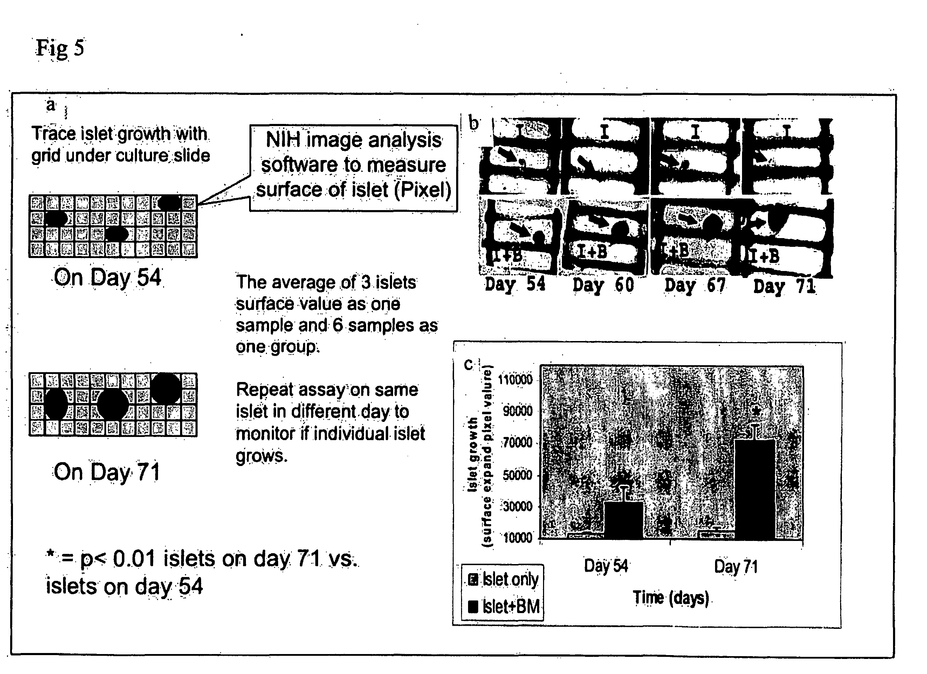

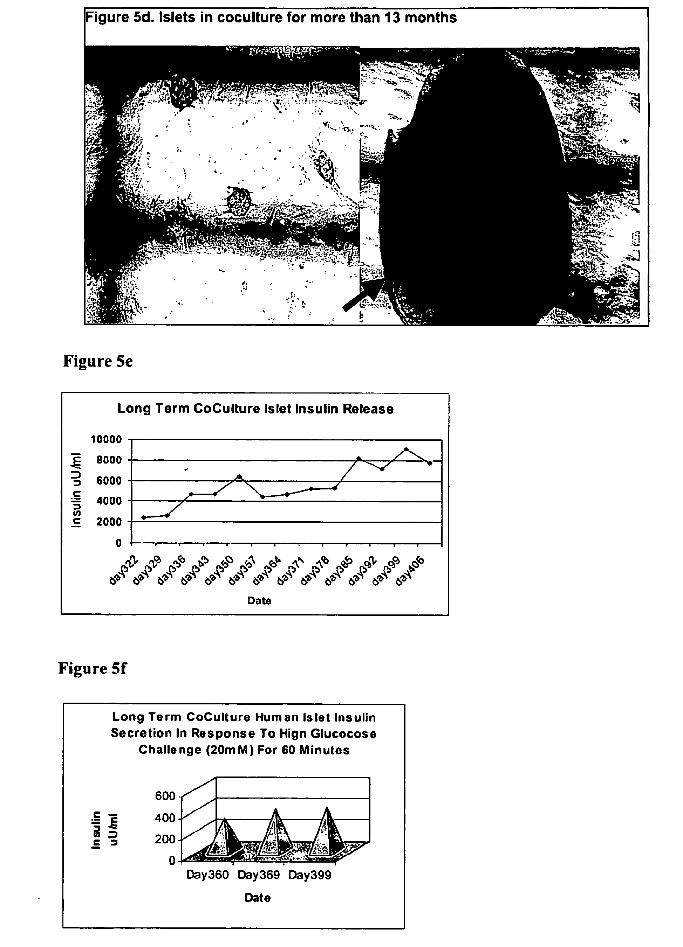

**Figure 5a**-**f**. Growth of islets in culture. Figure 5a is a schematic of the assay. Figure 5b shows islets grown on grids in the absence (top panel) or presence (bottom panel) of bone marrow cells. Figure 5c is a bar graph quantifying growth of islets in culture. (*= p< 0.05, n = 6). Figure 5d shows islets grown with bone marrow cells on a grid during neogenesis (left panel) or reinstitution (right panel at more than 13 months)(magnification 5x). Figure 5e is a graph of insulin release from the islet shown in the right panel of Figure 5d from day 322 to day 406 in culture. Figure 5f is a graph of insulin secretion in response to high glucose challenge on days 360, 369, and 399. Islet only cultures has no detectable insulin secretion at the same time points.

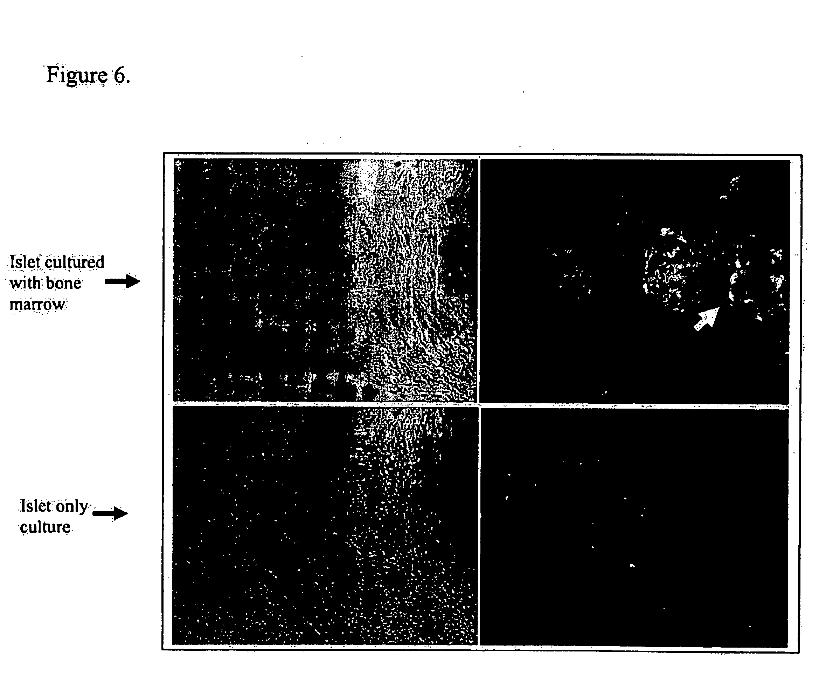

**Figure 6****..** Morphological and immunofluorescence analyses of islet β cells cultures with and without bone marrow cells using cell type specific markers at 153 days. Left panel images are phase contrast microscopy of islet β cells grown with (top panel) or without (bottom panel) bone marrow cells. Right panel images are fluorescent immunohistochemistry with antibodies to Ki67 and proinsulin (indicated by arrow). (Image magnification x 20).

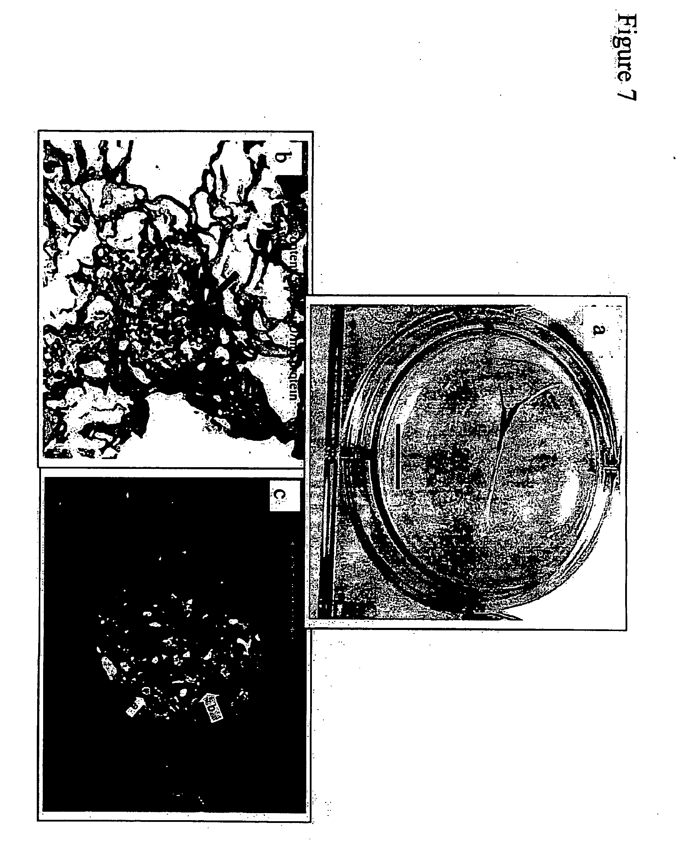

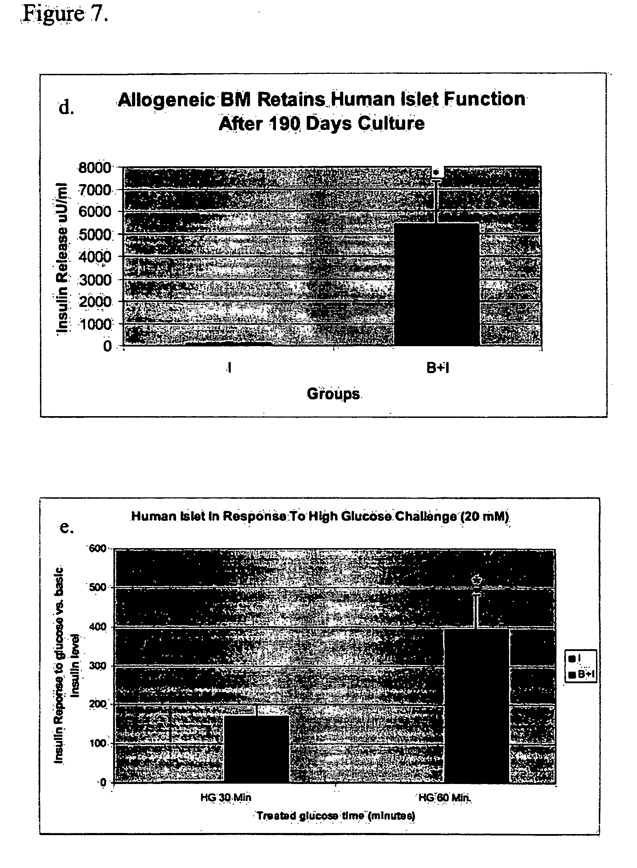

**Figures 7a****-e.** Morphological, immunofluorescence, and functional analyses of islet β cells cultures with marrow cells using cell type specific markers at 190 days. Figure 7a is an image of a tissue generated from islet cultured with bone marrow cells for 190 days, bar represents 1 cm. The tissue is about 1.8 cm long and has clear 3-dimensional structure. Figures 7b and c are 5 µm cross-sections from frozen tissue comparable to that shown in Figure 7a. Figure 7b shows histochemical staining with hematoxylin and eosin to reveal tissue structure. The arrow indicates islet like structure that appears in section surrounded by porous scaffold like tissue. Figure 7c, immunofluorescent staining with anti-human proinsulin antibody (indicated by the b arrow), a cell with anti-human glucagon antibody (indicated by arrow a) and nuclear staining DAPI. Figure 7d is a bar graph showing baseline insulin release from islet β cells in culture without (I) or with (B+I) bone marrow cells at day 190. Figure 7e is a bar graph showing insulin release in response to glucose challenge from islet β cells in culture without (I) or with (B+I) bone marrow cells on day 190. (p< 0.05 vs. islet only culture. Image magnification 20x)

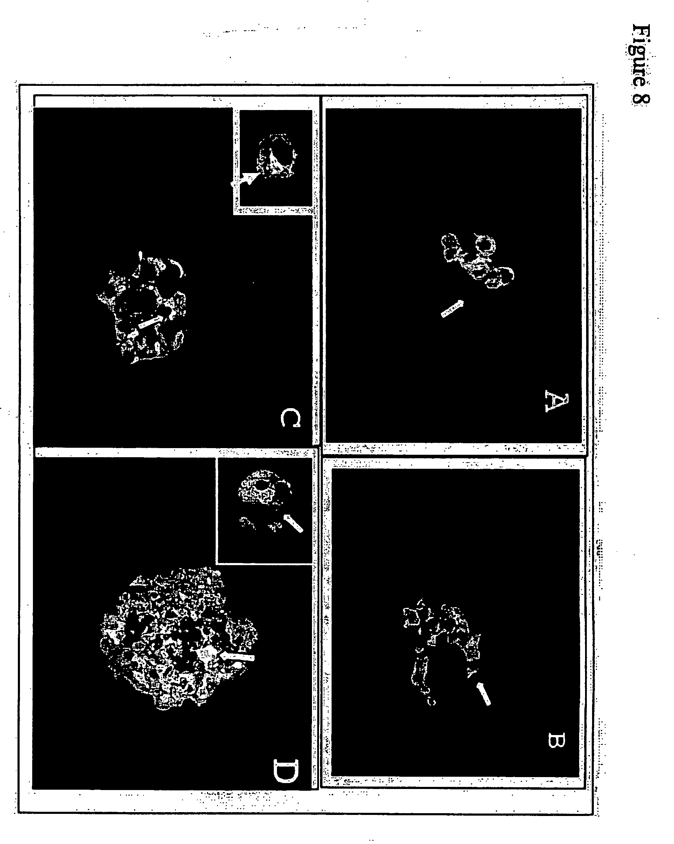

**Figure 8****.** Immunofluorescence analysis of islet β cells and bone marrow cells using live cell membrane dyes during the first 48 hours of co-culture. Human bone marrow cells labeled with PKI-26GL were co-cultured with islets stained with PK-2GL, and immunohistochemical staining was performed with specific anti-proinsulin antibody and DAPI for nuclear staining to study the interaction between bone marrow cells and islet β cells in culture. Figure 8a shows the cells after 7 hours. Figure 8b shows the cells after 24 hours. Figure 8c shows the cells after 48 hours. Figure 8d shows the cells after 96 hours. (Image magnification x 20, arrows indicate bone marrow and bone marrow colocalization with β cells)

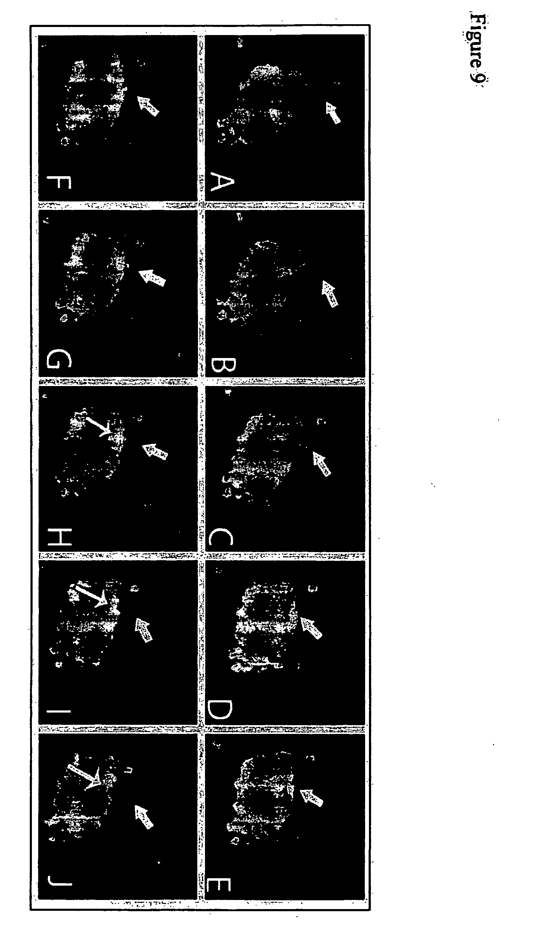

**Figures 9a**-**j**. Images from time-lapse microscopy were recorded at 20-minute intervals after introduction of bone marrow cells and islet β cells into co-culture. Bone marrow cells and islet cells were individually labeled with PKH26 and PKH 2 separately then cultured together. Images a-j are one episode from 96 hours culture. The thick arrow indicates bone marrow and thin arrow indicates bone marrow released materials in islet. (Image magnification x 20)

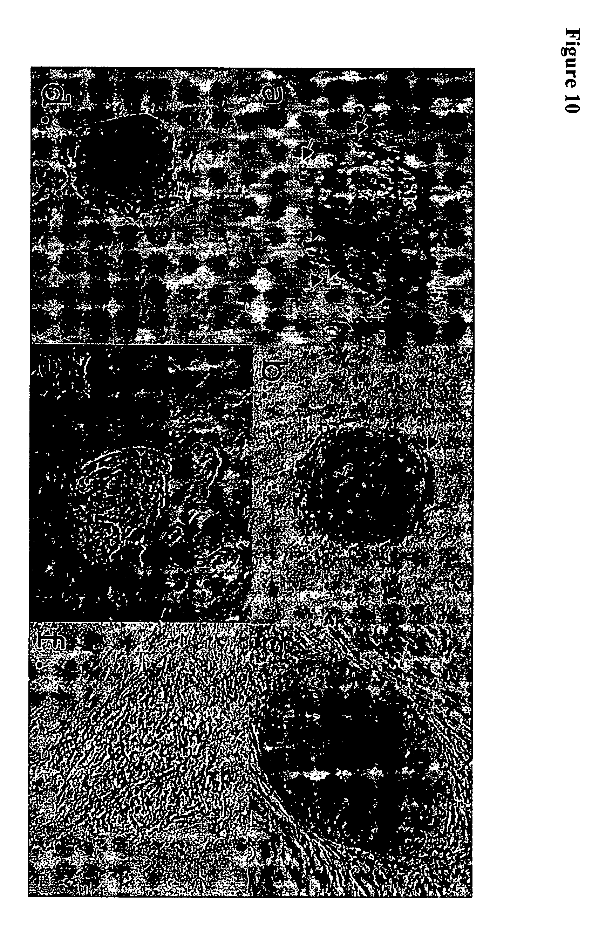

**Figures 10a****-f**. Images from time-lapse microscopy were recorded of three islet cells on a grid that migrate towards each other (Figures 10a-d) and finally fuse (Figure 10e). Figure 10f is from a separate experiment in which the bone marrow cells were labeled showing the migration of a bone marrow cell into the united islets.

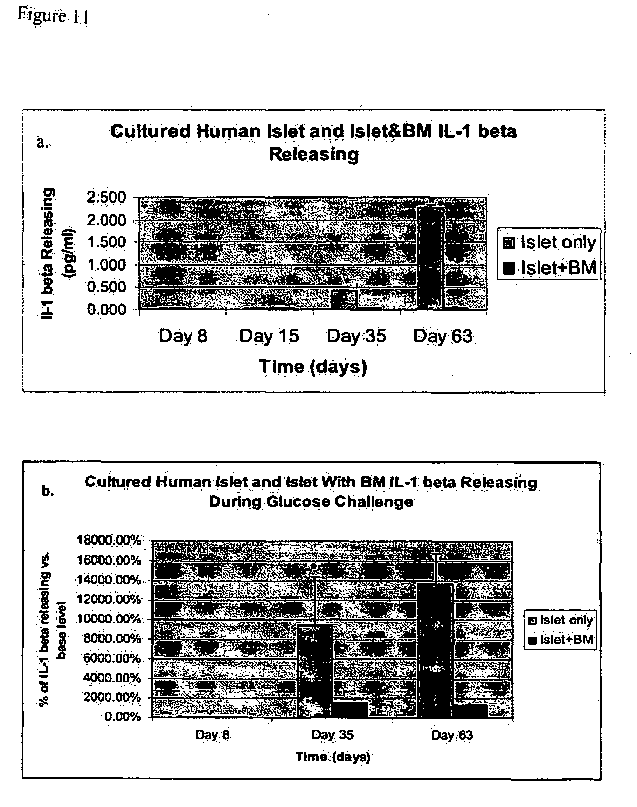

**Figure 11****.** Level of IL-1 β in culture medium of islet β cells cultures with marrow cells using cell type specific markers at various time points. Figure 11a is a graph showing IL-1 β release in culture. Figure 11b is a graph showing IL-1 β release in culture after high glucose (20 mM) stimulation. (n = 6, * = p< 0.001).

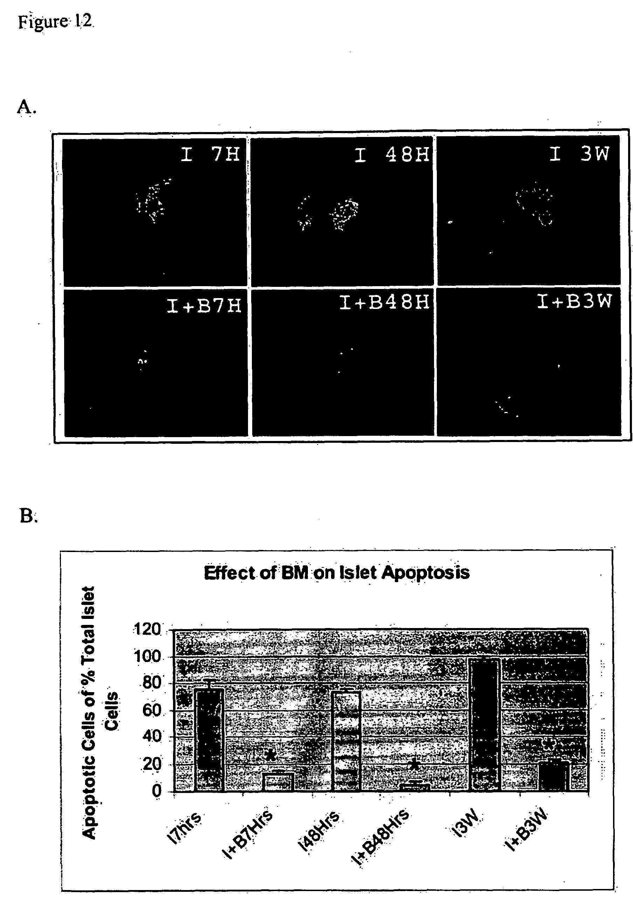

**Figure 12****.** Immunofluorescent analysis of apoptosis of islet β cells cultures with marrow cells using cell type specific markers at specific time points. Figure 12a shows immunofluorescent analysis of apoptosis in islet β cells in culture without (I) or with (B+I) bone marrow cells, as evaluated by (TUNEL) assay. Islet β cells are indicated by florescent immunohistochemical staining with a proinsulin antibody at 7 hours (7H), 48 hours (48H) and 3 weeks (3W) culture period. Figure 11b is a graph of a quantitative apoptotic cells versus total β cells in percentage at the times indicated. (n = 24, * = p< 0.01)

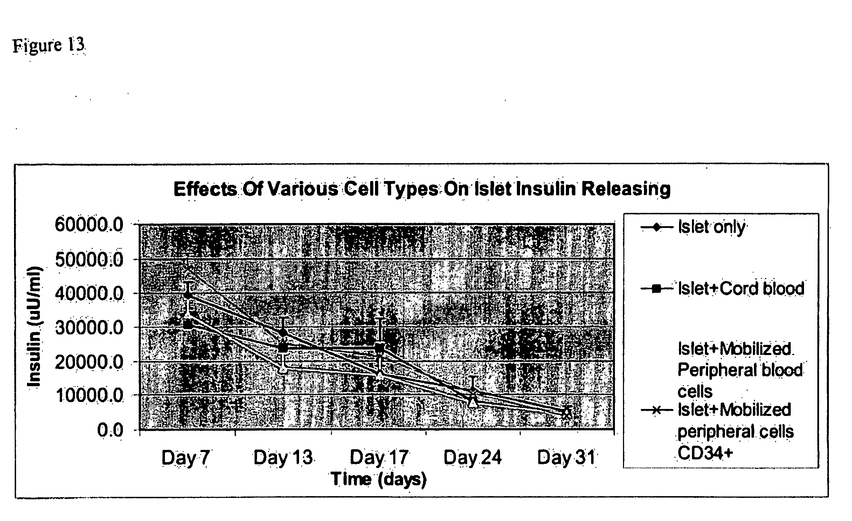

**Figure 13****.** Analysis of non-bone marrow cell types to support islet β cell function in culture. Figure 13 is a graph of insulin release from islet β cells co-cultured alone or with cord blood, peripheral blood, or peripheral blood CD34+ cells over a 31 day period.

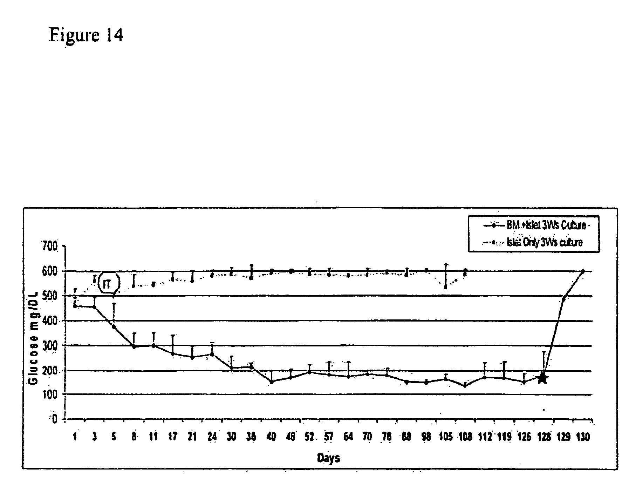

**Figure 14****.** *In vivo* evaluation of co-cultured islet β cells and bone marrow cells in a mouse model of diabetes. Figure 14 is a graph of blood glucose levels in mice transplanted with islet β cells that were cultured alone or islet β cells that were co-cultured with bone marrow cells.

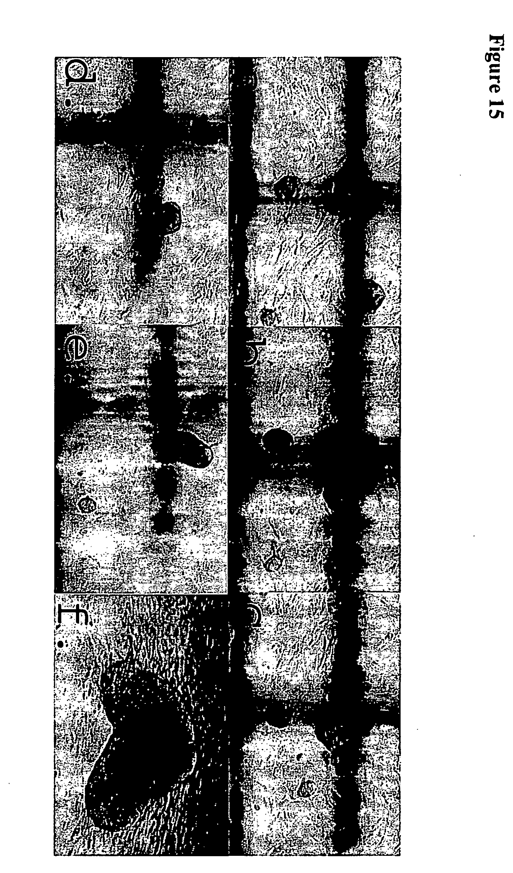

**Figure 15a-f.** *In vitro* demonstration of bone marrow facilitated migration of islet β cells. Time lapse microscopy images of formation of islet aggregates. Figures 15a-e magnification 5x. The dark color on the islet surface in Figure 15f indicates migration of the bone marrow cell into the islet. (magnification 20 x)

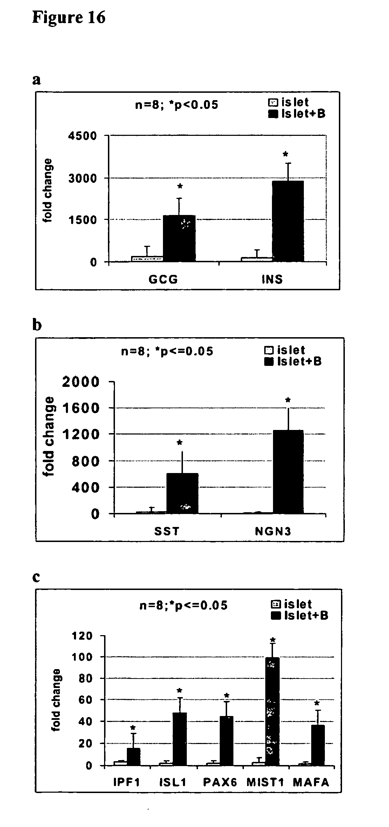

**Figure 16a****-c.** Figure 16a is a graph of fold change in expression level of GCG and INS in islet β cells grown without and with bone marrow cells. Figure 16b is a graph of fold change in expression level of SST and NGN3 in islet β cells grown without and with bone marrow cells. Figure 16c is a graph of fold change in expression level of IPF1, ISL1, PAX6, MIST1, and MAFA in islet β cells grown without and with bone marrow cells.

### Definitions

A "sustained period" is understood to be a period of time sufficient to preserve islet β cells such that the insulin releasing function of the cells is preserved, preferably with growth of islet tissue. According to the invention, the period is as at least about 30 days, preferably In certain embodiments, the period is as at least about 60 days, more preferably at least about 90 days, still more preferably at least about 120 days.

"Islet β cell function" can be defined by any of a number of assays including, but not limited to, morphological, immunohistochemical and functional assays. In a preferred embodiment, islet β cell function is defined by activity in an insulin release assay, either in resting cells or in response to glucose. In a more preferred embodiment, islet β cells are defined as having function by release of at least about 150 units of insulin over 30 minutes (in response to treatment with 20 uM glucose) in about 20 to 100 islets (IEQ). The exact method of determining islet cell function is not a limitation of the invention.

"Increased islet β cell viability" is understood as a lower rate of cell death as observed at a single time point or over time, preferably as compared to a control culture in which islet β cells are grown in the absence of bone marrow cells. The number of living cells as determined by methods known to those skilled in the art, such as trypan blue dye exclusion to identify viable cells, and cell counting using a hemocytometer to determine the number of living cells in a specific area or volume. Cell viability can also be determined using any of a number of commercially available apoptosis assays or by methods known to those skilled in the art. A lower percentage of apoptotic cells in a sample as compared to a control sample indicates increase islet β cell viability. Apoptotic assays can be combined with immunohistochemical staining to confirm the identity of islet β cells. The specific method of determining islet β cell viability is not a limitation of the instant invention.

"Islet" as understood herein is a clusters of cells present in the pancreas that secrete insulin and other hormones and form the endocrine portion of the organ, and are sometimes known as the islets of Langerhans. The islets constitute approximately 1 to 2% of the mass of the pancreas. There are about one million islets in a healthy adult human pancreas, which are interspersed evenly throughout the organ, and their combined weight is 1 to 1.5 grams. Each islet contains approximately one thousand cells and is 50-500 in diameter. Insulin-producing β cells constitute about 65-80% of the islet cells and glucagon-releasing alpha cells constitute about 15-20% of the islet cells. Islet cells also include somatostatin-producing delta cells (about 3-10%) and pancreatic polypeptide-containing PP cells (1%).

"Obtaining cells" is understood herein as manufacturing, purchasing, or otherwise obtaining cells.

The terms "expansion" and "expanding" as in "islet β cell expansion" are understood as increasing the number of viable and/or functional islet β cells in a culture over time. Expansion may occur by promoting cell division in existing islet β cells or conversion of bone marrow cells to an islet cell or both.

The term "in culture" can be understood to include the growth of cells, for example, in a Petri dish under appropriate conditions of temperature, CO₂ and nutrients. Cells can be cultured for example in the presence of matrices such as natural or artificial protein matricies from animals, plants, or seaweed. In the appropriate context, in culture can be understood, for example, to grow under non-native conditions of the cell. For example, cells can be grown in culture implanted in an animal such as a mouse, including in a specific organ of an animal, or in an *ex vivo* organ culture prior to transfer into an animal.

The terms "preserve" and "preserving" as in "preserve or preserving islet β cell function" are understood to include an increase in islet β cell viability, and/or an improvement in islet β cell function, and/or an improvement in islet β cell morphology as compared to an islet β cell cultured in the absence of a bone marrow cell.

The term "subject" refers to living organisms. In certain embodiments, the living organism is an animal. In certain preferred embodiments, the subject is a mammal. In certain embodiments, the subject is a domesticated mammal. In certain embodiments, the subject is a human. Subjects include humans, monkeys, dogs, cats, mice, rats, cows, horses, goats, and sheep. The subject may be diagnosed with diabetes. In other embodiments, the subject has been diagnosed with some other pancreatic.

The terms "selecting a subject" or "identifying a subject" are understood as choosing one or more members of a mixed population of individuals based on specific characteristics including, but not limited to, physical symptoms, clinical characteristics as determined by diagnostic methods.

The term "monitoring a subject" is understood as observing a subject after implantation of islet cells, preferably both before and after implantation if islet cells, for altered islet cell function, preferably improved islet cell function.

Ranges provided herein are understood to be shorthand for all of the values within the range. For example, a time of 1 to 50 seconds is understood to include 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 seconds.

Unless specifically stated or obvious from context, as used herein, the term "or " is understood to be inclusive.

Unless specifically stated or obvious from context, as used herein, the terms "a", "an", and "the" are understood to be singular or plural.

### DETAILED DESCRIPTION AND PREFERRED EMBODIMENTS

In this study, the ability of allogeneic bone marrow to support donor human islet's endocrine function *in vitro* was explored. Co-culture of human islets P cells with allogeneic bone marrow was shown to increase islet survival and function with the eventual formation of new pancreatic endocrine tissue capable of sustaining the microenvironment to retain islet structure and β cell function.

Bone marrow cells were shown to be capable of stimulating pancreatic endocrine tissue growth while reducing release of inflammatory cytokines, such as IL-1β, from the islet cells. Co-culture with bone marrow cells also reduced islet cell apoptosis. Bone marrow cells were eventually shown to reconstitute human islet cells into functional pancreatic endocrine islet tissue under long-term culture (over 13 months). These effects were found to be bone marrow specific. Cord blood cells and/or isolated peripheral blood CD34⁺ cells were of no benefit in the maintenance of islet function or survival *in vitro.* This demonstrates that bone marrow offers a unique advantage in the support of human endocrine pancreatic tissue that may significantly improve the success in achieving insulin independence.

The invention includes co-culture of an islet β cell with a bone marrow cell. However, in a preferred embodiment the ratio of about 25 to 100 islets to about 1-5 x 10⁶ bone marrow cells. It is understood that other ratios of islets to bone marrow cells is possible, and that the culture may include additional cell types such as bone marrow stromal cells, mesenchymal cells, and pancreatic alpha cells.

Although not wishing to be bound by mechanisms of action, the data suggest that bone marrow cells increase the morphology, viability, and/or function of islet cells by reducing cytokine release from the islet cells including IL-1β. This is proposed to result in a reduction of inflammatory factor release, and a reduction in apoptosis or cell death. Co-culture was also demonstrated to increase the expression of pancreas specific genes and growth factors associated with regeneration. The data disclosed herein suggest that although some marrow cells take on the phenotype of β-cells, it is probably a minor component of the demonstrated survival and growth effects. It is more likely that the demonstrated anti-apoptotic effects of the co- culture methods of the invention are based upon paracrine effects, one of which may be the suppression of the inflammatory cytokine, interleukin-1β [25, 28-30]. The islet cell proliferation and growth stimulated by bone marrow paracrine growth factors is likely to be a major factor to contribute to reconstitute islet tissue [20, 31-33].

The invention is not limited to the co-culture of islet β cells with complete bone marrow. Methods of cell sorting by gradient, cell surface markers, and other methods are well known to those skilled in the art. It is expected that one or more subpopulations of bone marrow cells can be effective in promoting islet β cell viability, morphology, and function over a sustained period in culture. The method is also not limited by the source of cells. It is understood that the use of the method of co-culture of allogeneic islet β cells and bone marrow cells can be used for the culture of any mammalian islet β cells and bone marrow cells. The term "mammal" is used herein to refer to a warm-blooded animal such as a rodent, rabbit, or a primate and especially a human patient. Specific rodents and primates of interest include those animals representing accepted models of human disease including the pig, chimp, sheep, goat, horse, mouse, rat, rabbit, and monkey. Particular human patients of interest include those who have, are suspected of having, or are at risk of having a decrease or loss in pancreatic cell function. Moreover, methods including the culture of human islet cells in bone marrow from nude mice or other mammals lacking an immune system is within the scope of the instant invention.

The data disclosed herein indicate that co culture of allogeneic human marrow with β-cell islets allows for preservation of islet numbers and insulin releasing function with growth of islet tissue for a sustained period, including over 13 months *in vitro.* In previous studies cultured human islets have generally lost function and viability after 3-4 weeks [27]. Cells in the islets in these studies include α and β cells with preservation of baseline insulin production and response to a glucose challenge. These observations suggest immediate strategies for islet cell transplantation using co-culture with allogeneic bone marrow cells. However, the methods of the invention are not limited by the use of the islet cells after their growth in culture.

The present invention is further illustrated by the following examples. These examples are provided to aid in the understanding of the invention and are not construed as a limitation thereof. The contents of all references, patents and published patent applications cited throughout this application, as well as the figures, are incorporated herein in their entirety by this reference.

### EXAMPLES

### Example 1- Cell staining methods

Methods of cell staining and immunofluorescence using fluorescent and chemical dyes is well known to those skilled in the art. A number of examples include such staining methods. An exemplary method of cell staining is provided. Cells grown on chamber slides were fixed with 3% paraformaldehyde, followed by exposure to 10% normal goat serum. The slides were blotted without washing and a mixture of the primary antibodies were applied, and the slides were then incubated in a moist chamber at 4 °C overnight. The slides were washed 3 times, followed by exposure to the secondary antibody, for 45 minutes, at room temperature. After washing, diluted secondary antibody was applied and the slides were incubated for 15 minutes. The slides were subsequently washed extensively with PBS and the above process optionally repeated with a second fluorescent color (e.g., DAPI) and/or third antigen-detecting antibody. When the process was finished, the slide was covered with fluorescent mount medium and a cover slide. The samples were then observed and photographed using a confocal fluorescence microscope [22].

### Example 2- Co-culture of human bone marrow and human islet β cells

Human islet tissue, from normal donors, was obtained from Islet Resource Centers (ICRs) in the ICR Basic Science Islet Distribution Program, Human Islet Laboratory, University of Pennsylvania (Philadelphia, PA), Joslin Diabetes Center

(Boston, MA) and City of Hope National Medical Center (Duarte, CA). The use of these cells were approved by the IRB at Roger Williams Hospital and the ICRs Committees.

Human bone marrow from normal donor was obtained after signing the appropriate consent form that had been approved by Roger Williams Hospital Institutional Review Committee (IRB). Bone-marrow mononuclear cells were isolated by Ficoll-Paque™ Plus (Amersham Biosciences; Amersham, UK) per manufacturer directions. Cells were then washed twice with 5% FCS/PBS, resuspended in culture medium (see below). Trypan blue staining was used to assess cell viability.

Human islets were received from Islet Cell Resource Centers (ICRs) within 48 hours after death of voluntary donors (with purity of > 90%, viability > 95%). Fifty islet equivalents (IEQs) per ml with 1 x 10⁶/ml allogeneic whole BM cells were cultured in RPMI 1640 (manufacturer) supplemented with 10% heated inactivated Fetal Bovine Serum (HiFBS), 5.5 mM glucose, 10 mM HEPES, and 1% P/S in a humidified 37°C incubator, 5% CO₂.

Figures 1a-d show islet β cells grown in the absence (a and c) or presence (b and d) of bone marrow cells for either 6 (a and b) or 156 (c and d). A difference in morphology is notable after only six days. The cells grown in the absence of bone marrow cells began to form a monolayer (Figure 1a), whereas the cells grown in the presence of bone marrow cells did not show a similar loss of morphology. Bone marrow cells surround the islet cells to protect the islet cell membranes and prevent the cells from spreading (Figure 1b). The difference in morphology was even more striking on day 156. Cells grown in the absence of bone marrow cells formed a monolayer (Figure 1c), whereas those grown in the presence of bone marrow cells formed a round islet (Figure 1d).

Figures 2a-b show islet β cells grown in the absence (left panels) or presence (right panels) of bone marrow cells on day 28. Islets were digested with 0.05% trypsin (Promega, Madison, WI) for 5 minutes. After one wash with PBS, cytospin slides of the cells were made. The cells in the top panels of Figure 2a were stained with DAPI to visualize the nuclei. The cells in the bottom panel were stained with antibodies targeted to proinsulin and CD 45, a bone marrow cell marker, and visualized with secondary antibodies that appear as different colors upon imaging.

The cell number was substantially less in the islet cell alone culture (left panel) as compared to the bone marrow co-culture (right panel). Moreover, in the islet cell only culture, a number of cells did not appear to be expressing proinsulin (i.e., are stained with DAPI, but not with the anti-CD45 antibody).

In the right hand panels, the relatively large cluster of cells indicated by the thicker, lower arrow were stained for proinsulin with some speckled CD45 staining suggesting the presence of some bone marrow cells in the cluster. The thinner, upper arrow indicates a cell that was stained with the CD45 antibody. The majority of cells not staining for proinsulin in the co-culture stained for CD45, indicating that they are bone marrow cells.

To confirm the observations from the immunofluorescent analysis, cells were stained using immunohistochemical methods. Proinsulin expressing cells and CD 45 expressing cells were stained with distinct chromophores. Again, a number of the islet cells in the bone marrow alone culture were not expressing proinsulin, whereas almost all of the non-bone marrow cells in the co-culture were expressing insulin. Proinsulin staining is indicated by the arrow in the left panel, and the arrow further to the right in the right panel. CD45 staining is indicated in the left panel by the arrow further left in the panel. Again, more viable cells and more proinsulin staining were observed in the co-culture.

A quantitative analysis of the percent of proinsulin positive islets is shown in Figure 1c. A significantly larger number (2.7 fold higher) of islet cells in the co-culture were found to express proinsulin as compared to the islet cell alone culture (p < 0.01, n = 6)

Figure 3 shows an islet that was formed in culture at 153 days. The left panel is a phase contrast image of the islet. The center and left panel are confocal images at the top and center of the islet, respectively. The α cells were stained with antibodies to glucagon and indicated by the arrow towards the center of the cell mass and β cells were stained with antibodies targeted to proinsulin and are indicated by the arrow towards the periphery of the cell mass. Alpha cells release glucagon in response to low blood sugar. As can clearly be seen in the confocal images, the β cells surrounded the α cells. This analysis demonstrated that β cells represent the majority of the cell population, and α cells are localized in the central portion of the islet, suggesting that a reconstitution occurred in these cultures [23, 24]

These results clearly demonstrate that bone marrow cells can serve to maintain the morphology of pancreatic islets and maintain the expression of islet cell specific markers *in vitro* for a sustained period.

### Example 3- Evaluation of islet function in an insulin release assay

Islet cell function was evaluated by measurement of insulin release with or without a glucose challenge. The culture media was collected twice per week and stored at -80°C until assay for the basal insulin release by ELISA.

A high-glucose challenge assay was performed once a week as follows: Media was collected, and cultured cells were washed once with RPMI medium. The media was then replaced with high-glucose (20 mM) RPMI 1640 for 15 and 30 minutes. The media was finally collected and stored at -80°C until insulin assay by ELISA.

Insulin concentrations in the specimens (cell culture medium or tissue extracts) were measured using Human Insulin ELISA Kit (Linco Research, St. Charles, MO) according to the manufacturer's instructions. Briefly, insulin standards and appropriately diluted (1:50-1:500) samples were added to an insulin antibody-coated 96-well microplate and incubated for 2 hours at 4 °C. After washing, anti-human insulin enzyme conjugate was added to each well and incubated for 30 minutes at room temperature. After washing, enzyme substrate solution was added and then incubated for 45 minutes at room temperature in the dark. Reactions were stopped by adding 1N sulfuric acid. Absorbance at 450 nm was read with µQuant^{™} microplate reader (Bio-Tek Instruments, Inc., Winooski, VT) and concentrations were calculated by KC Junior^{®} microplate reader software (Bio-Tek Instruments, Inc.) [20].

The functional integrity of the cells was determined using a basic insulin release assay and an insulin secretion in response to high glucose challenge assay. These assays demonstrated that islets co-cultured with bone marrow cells released insulin in a stable manner for 204 days in culture (Figure 4a). Insulin release levels were nearly stable at about 8000 µU/ml at the beginning to about 10,000 µU at 195 days of culture. Increased human insulin release over time suggests regeneration and/or expansion of islet cells over time.

Islets cultured alone revealed unstable insulin release (Figure 4a), beginning with an average of about 9,500 µU/ml with significant variation from a high of about 17,500 rapidly declining to about 4,900 µU/ml at 28 days of culture, and loss of function by the fifth week. In conjunction with morphological studies, these findings the spikes at days 15 and 28 were likely due to leakage of insulin from dying β cells within the islets cultured alone. In a similar fashion, the relatively low basal insulin release noted in the first 21 days of the co-culture may have been due to a paucity of dying β cells prior to the achievement of functional stabilization.

Evaluation of insulin secretion in response to glucose challenge (20 mM) revealed that islet cells cultured with bone marrow cells are able to respond to glucose for 204 days in culture as opposed to islet cell alone cultures that lose their ability to respond to a high glucose challenge largely by day 42 and completely by day 70 (Figure 4b). The difference between islet only culture and islets with BM, in regards to insulin release and insulin in response to glucose challenge, was statistically significant (p < 0.01, n = 6).

### Example 4- Co-culture of islet β cells with bone marrow cells promotes cell growth and islet mass formation

Cell growth and islet cell formation was monitored by growing islet β cells without or with bone marrow cells on grids. A schematic of islet cells on a grid are shown in Figure 5a. Using the grid, single islets can be monitored over time for growth. Islet cells were traced and morphological changes recorded as photomicrographs. NIH image analysis software was used to quantitate islet surface area (Pixel value). The average of three islets surface values was considered as one sample, and six samples were collected per group.

In the first three weeks of cultures, despite significant functional differences, little difference in morphology was observed between the islets that were cultured with and without bone marrow cells (not shown). However, after three weeks the islets cultured in the presence of bone marrow cells continued to expand, while islets cultured in the absence of bone marrow cells, shrunk and they eventually disappeared (Figure 5c). The change in islet and cell size were quantitated using image analysis software. The results demonstrated that bone marrow co-culture with islets resulted in an expansion of their surface by 1.8- fold during a 17 day period (from day 54 to day 71). This was significantly different compared to islet only cultures where the cells showed essentially no increase in size and no islet formation (Figure 5c, p< 0.05, n = 6).

Figure 5d shows growth of the islet cells early in neogenesis (left panel) in co-culture for 13 months (right panel) on the gridded slide. The substantial increase in size of the islet over time in the co-culture is easily observed on the gridded slide. The increased islet size may result by stimulation of islet proliferation by bone marrow cells which identified by their significant Ki67 expression (Figure 6).

Figures 5e and f demonstrate that the islet cells remained functional after 13 months in culture. Functional assays were performed as above. Insulin release was observed through the end of the observation period of 406 days (Figure 5e). Insulin secretion in response to high glucose was observed on days 360, 369, and 399 as shown (Figure 5f). These data demonstrate sustained function of the islet β cells grown in the presence of bone marrow for at least 13 months.

### Example 5- Co-culture of islet β cells with bone marrow cells promotes proliferation

Figure 6 shows islet β cell cultures grown with (top panels) or without (bottom panels) bone marrow cells after 153 days in culture. The left hand panels are phase contrast images. In the right hand panel, the cells are stained with proinsulin and Ki67, a marker for cell proliferation. Nuclei are visualized with DAPI. The top panels show that the cells have developed an islet morphology by the large clusters of proinsulin staining cells and are proliferating as demonstrated by substantial proinsulin staining. Many spots of Ki67 staining are also observed, including the cell indicated by the arrow. The bottom panels show a relatively homogenous monolayer with almost no proinsulin or Ki67 staining. In the islet cultures the cells are visualized essentially only by DAPI staining. These images demonstrate that islet β cells cultured in the presence of bone marrow cells express proinsulin, form islets, and proliferate for a sustained period in culture. Conversely, no specific islet morphology was observed in the islet β cell culture alone. Similarly, almost no proinsulin or Ki67 staining was observed in the islet β cell culture alone.

### Example 6- Co-culture of islet β cells with bone marrow cells promotes the formation of macroscopic islets

After long-term culture of islet β cells with bone marrow cells, macroscopic tissues were formed. Figure 7a displays a tissue generated from islet cells cultured with bone marrows cells for 190 days. The bar represents 1 cm. The tissue is about 1.8 cm long with clear 3-dimensional structure. Five micron frozen sections were prepared from a comparable tissue sample and stained with hematoxylin and eosin to reveal the morphology of the tissue (Figure 7b). Histological staining showed that the tissue had an islet-like structure (arrow indicated) that appears in the section, surrounded by porous scaffold like tissue. The insulin content of the tissue was found to be 56307 uU/per mg protein (ELISA assay). The islet like tissue contained predominantly β cells and a few α cells as show by fluorescence immunohistochemistry (Figure 7c). Islet β cells were stained with antibodies to proinsulin (indicated by arrow a), α cells with antibodies to glucagon (indicated by arrow b), and nuclei were stained with DAPI.

The tissue was analyzed for both basal insulin release and induced insulin secretion in response to high glucose using the methods described above. As expected, the tissue formed in the islet β cell-bone marrow co-culture demonstrated both basal insulin release (Figure 7d) and induced insulin secretion in response to high glucose (Figure 7e). Essentially no insulin secretion was observed from cells in an islet β cell only culture of the same age. These data demonstrate that co-culture of islet β cells with bone marrow cells allows for the formation of higher order three-dimensional structures that have islet-like structure and function.

### Example 7- Interaction of bone marrow cells with islets cell co-cultures

The ability of bone marrow cells to preserve islet β cells in culture was further demonstrated using histochemical and immunofluorescence analyses. A PKH cell labeling kit (Sigma, St. Louis. MO) was used to label bone marrow and islet β cells for monitoring the interaction between the cell types. PKH dyes can be used for live cell labeling without affecting the morphology or function of cells. The dyes are fluorescent labels attached to long lipophilic tails that integrate into the membrane, leaving the fluorogenic moiety exposed near the surface of the cell. The dyes allow for the tracking of two types of live cells in a population. Following the manufacture's instructions, 4 µM of PKH 26 (PKH26GL) and PKH 2 (PKH2GL) were incubated with 1 x 10⁶ bone marrow cells and 50 islets for 3 minutes, respectively. Cells were washed separately in PBS. Staining was stopped by adding 1 ml cold 1% BSA in PBS. Cells were gently pelleted and resuspended in PBS five times to remove any unbound dye. After labeling was complete, bone marrow cells and islets were co-cultured in under the conditions described above.

The cells were collected after 7, 24, 48 and 96 hours in culture. Cells were stained with an antibody targeted to proinsulin. It was observed that bone marrow cells (thick arrow) closely approached the islets at 7 hours (Figure 8a) and actively migrated into the islets by 24 hours (Figure 8b). After 48 hours, the bone marrow cells appeared to adhere to the P cells within the islet (Figure 8c, inset high magnification). A high magnification image showed bone marrow cells interacting with islet β cells. Furthermore, bone marrow cells appeared to foster the coalescence of the β cells together to form entire islets as seen in the photomicrograph, at 96 hours of culture, as shown in the top left corner high magnification image (Figure 8d, inset high (magnification). One may note the bone marrow associating with islet β cells at either side.

The interaction of bone marrow cells with islet β cells was also analyzed utilizing real time Time-Lapse Microscopy and vital dyes to monitor bone marrow cells and human islets in culture for 64 hours, with an image recorded every 20 minutes (Figure 9). A bone marrow cell (thick arrow) moved progressively closer to the islet as seen in Figure 9a, b, and c. This was followed by entrance of the bone marrow cell into the islet cluster, as seen in d, e, and f. The bone marrow cell gradually moved out from the islet as seen in g, h, i, and j (thin arrow). The images clearly demonstrate that BM cells make contact with the islet cells and release materials into the proximity of the islet cells.

Migration of bone marrow cells in culture with islet β cells was also observed over longer periods to provide insight into how bone marrow participates in islet regeneration. In Figure 10, human bone marrow cells labeled with the vital dye PKH-26 (indicated with arrows) separated from human islet (large cluster) at 3 hours' coculture (Figure 10a) migrated into islet after 30 days' culture (Figure 10b), and formed a capsule-like tissue surrounding islet at 60 days' culture (Figure 10c). Islet-only culture did not show any red at 3 hours (Figure 10d), and islets became monolayer in culture on day 30 (Figure 10e) and remained so at day 60 (Figure 10f). (magnification x 20)

### Example 9- Co-culture of islet β cells with bone marrow cells inhibits cytokine release

It has been known that IL-1 β, produced and released from human islets, plays a critical role in islet survival and function [25, 26]. To evaluate the role of IL-1β in the survival of islet cultures, IL-1 β levels were measured in islet β cell cultures in the presence and absence of bone marrow cells. Using an ELISA kit specific for human IL-1 β, a gradual increase of IL-1β release from the islets was demonstrated when the islets were cultured alone. The level of the cytokine production increased from non-detectable levels at the seeding of cultures to 25 fold increases after 63 days of culture. This is in contrast to islets cultured with bone marrow cells where IL-1 β levels were very low (Figure 11a). Further evaluation of IL-1 β release in the presence of a high glucose challenge indicated that the level of IL-1 β release increased 1500 fold in islets cultured alone while the IL-1 β levels remained very low when the islets were cultured with bone marrow (Figure 11b).

### Example 10- Co-culture of islet β cells with bone marrow cells promotes cell survival

Islet β cell apoptosis is recognized as the major cause for β cell loss *in vivo* and *in vitro,* whether bone marrow regulation of human islet survival and function is related to the cultured islet apoptotic process was analyzed. A terminal deoxyribonucleotidyl transferase (TDT)-mediated dUTP-digoxigenin nick end labeling (TUNEL) method (Clone ApoAlert^{™} DNA Fragmentation assay kit BD Clontech) was used to detect apoptotic nuclei according to the manufacturer's instructions. Briefly, human islet preparations were fixed in 3% paraformaldehyde and incubated in 0.3% H₂O₂ in methanol for 5 min to block endogenous peroxidase. Slides were incubated with TdT incubation buffer for 2 hours at 37 °C and terminated with 2 x SSC. Cells were also stained with an anti-human proinsulin antibody. The apoptotic cells were analyzed by calculating the total number of apoptotic cells divided by the total number of insulin positive cells without prior knowledge of the treatment protocols. Results were calculated from the measurement of more than 10 islets picked randomly from each slide and a total four slides were measured from each sample [21].

Examination of apoptosis as a function of time from the initiation of culture revealed, as early as 7 hours, that bone marrow cells protect islets from apoptosis and that the effects were significant when compared to islets grown in the absence of bone marrow (Figures 12a and b). Figure 12a shows substantial TUNEL staining beginning at 7 hours in the islet only (I) culture (top row). Protection of islet cell apoptosis by bone marrow cells becomes more evident as the duration of the culture increases, e.g., comparing the cells at 48 hours and at 3 weeks. Although some non-apoptotic cells could be observed at 8 hours, almost none could be identified at 3 weeks in the islet only culture image. This was clearly distinct from the islet + bone marrow (I + B) culture in which the cells appear to be substantially non-apoptotic at all time points observed (bottom row). Results from the experiments were quantified and the percent of apoptotic cells in the cultures is shown in Figure 12b and reflects what is shown in the images demonstrating an almost immediate protective effect of bone marrow on the islet cells.

### Example 11- Co-culture of islet β cells in the presence of cord blood cells or CD 34+ peripheral blood cells.

To evaluate cells besides BM for their effect on islet function, whole umbilical cord blood cells, mobilized peripheral blood cells, and mobilized peripheral CD 34+ cells were cultured with islets under the same conditions as the BM culture described above to determine if they could support islet β cell growth and promote islet formation. After the indicated number of days in culture, insulin release was analyzed (Figure 13). Data from islet insulin release measurements show no significant difference between these cells and islet-only culture, which indicates that BM cells may offer a unique advantage in islet cell survival.

### Example 12- Reconstitution of pancreatic function in a mouse model of diabetes using islet cells co-cultured with bone marrow cells.

To evaluate of the islet cells co-cultured with bone marrow cells could function in a pancreatic islet cell transplant method in a mouse model of diabetes, islet cells maintained in culture for three weeks either alone or in co-culture with bone marrow were used for islet cell transplant. Mice (8-week old, male Balb/c SCID mice) were injected with STZ 200 mg/kg body weight (i.p.) to induce diabetes as defined by having a blood glucose level in excess of 400 mg/dL consistently for three days. Human islet cells cultured without (I3W= 1500 islets (IEQ)) or with bone marrow (I3W= 1500 islets (IEQ) with 1 x 10⁶ bone marrow cells) were implanted into the left, subrenal capsule. Blood glucose levels were monitored (Figure 14).

After four months, animals transplanted bone marrow co-cultured islet cells had normal blood glucose levels and blood had detectable human insulin levels (157 uU/ml). Animals transplanted with islet cells that were grown in culture alone remained hypoglycemic. These data strongly suggest that the pancreatic insulin response is replaced by the transplanted islets grown in the bone marrow co-culture, but not by the islets grown in the islet only culture.

To insure that glycemic control was induced by the transplanted cells, the kidney containing the transplanted cells was removed. In response, the animal became hyperglycemic. These data demonstrate the utility of islet cells co-cultured with bone marrow cells for use in pancreatic islet cells transplant and for the treatment and/or amelioration of diabetes.

### Example 13- Co-culture of islet β cells with bone marrow cells facilitates islet aggregation

Bone marrow facilitates islet aggregation. Previous data has shown that substantial pancreatic tissue forms in coculture, which may result from bone marrow stimulation of islet aggregation. Data from a sequence of time-lapse microscopy images (Figure 15) indicate that bone marrow stimulates the process of islet aggregation and the formation of unified pancreatic tissue. The use of a grid under culture enabled identification and monitoring of individual islet migration in live microscopic images taken once a week.

In Figure 15a three islets are visible in certain areas of the grid. Two of the three bigger islets migrate towards each other in Figures 15b, c, and d, and finally unite to form a larger islet in Figure 15e (magnification = x 5). After labeling bone marrow with the vital dye PKH-26 (in a separate experiment), bone marrow was found to migrate into the united islets and formed an encapsulated, reconstituted islet (Figure 15f) (magnification = x 20).

### Example 14- Analysis of gene expression in islet cells in culture in the absence and presence of bone marrow

Gene expression was analyzed in human islet β cells grown in islet alone (islet) or islet and bone marrow co-cultures (islet + B). Expression levels of endocrine cell-specific gene expression and regeneration transcription factor genes was analyzed using RT-PCR with gene specific primers. Cells were cultured for 209 days, total RNA was isolated and reverse transcribed, and the resulting cDNA was subject to amplification by PCR using routine methods.

Expression of endocrine specific genes GCG, INS, and SST were significantly increased as was expression of the regeneration associated gene NGN3 in the cells in co-culture, but not in the islet only culture (Figures 16a and b). These results suggest that bone marrow cells stimulates human islet cell regeneration *in vitro.* Similarly, an increase in expression of the pancreas specific transcription factors PDX1 or IPF1, PAX6, ISL1 and MAFa was also seen in the co-cultures, but not in the islet alone culture (Figure 16c). These transcription factors regulate migration, differentiation, and proliferation in culture that may result in the enhancement of long term survival and function observed in the co-cultures.

Mist1 is a basic helix-loop-helix transcription factor that is specifically expressed in exocrine cells and is necessary for the organization and function of pancreatic acinar cells. Studies suggest that Mist-1 expression provides a microenvironment for support of islet development and function. Expression of Mist1 may allow for organization of the islet cells into higher order structures

### Statistical evaluations:

All data are presented as the mean +/- SEM and analyzed by the Analysis of Variance (ANOVA) followed by a student-t test, unless otherwise indicated. Sigma Plot (SPSS Inc. Chicago, IL) was used to draw charts. All data represents the results of three

### REFERENCES

1. Yamada, S. and I. Kojima, Regenerative medicine of the pancreatic beta cells. J Hepatobiliary Pancreat Surg, 2005. 12(3): p. 218-26.
2. Das, S.R., et al., Increased cardiovascular risk associated with diabetes in Dallas County. Am Heart J, 2006. 151(5): p. 1087-93.
3. Borrero Pachon, M.P., [Diabetic foot]. Rev Enferm, 2006. 29(3): p. 8-14.
4. Balamurugan, A.N., et al., Prospective and challenges of islet transplantation for the therapy of autoimmune diabetes. Pancreas, 2006. 32(3): p. 231-43.
5. Shapiro, A.M., et al., International Trial of the Edmonton Protocol for Islet Transplantation. N Engl J Med, 2006. 355(13): p. 1318-1330.
6. Lakey, J.R., M. Mirbolooki, and A.M. Shapiro, Current status of clinical islet cell transplantation. Methods Mol Biol, 2006. 333: p. 47-104.
7. Kayed, H., et al., Hedgehog signaling in the normal and diseased pancreas. Pancreas, 2006. 32(2): p. 119-29.
8. Lingohr, M.K., et al., Specific regulation of IRS-2 expression by glucose in rat primary pancreatic islet beta-cells. J Biol Chem, 2006. 281(23): p. 15884-92.
9. McCabe, C., A. Samali, and T. O'Brien, Cytoprotection of beta cells: rational gene transfer strategies. Diabetes Metab Res Rev, 2006. 22(3): p. 241-52.
10. Truong, W., et al., Coinhibitory T-cell signaling in islet allograft rejection and tolerance. Cell Transplant, 2006. 15(2): p. 105-19.
11. Matsuda, T., et al., Inhibition of p38 pathway suppresses human islet production of pro-inflammatory cytokines and improves islet graft function. Am J Transplant, 2005. 5(3): p. 484-93.
12. Chae, H.Y., et al., Effective glycemic control achieved by transplanting non-viral cationic liposome-mediated VEGF-transfected islets in streptozotocin-induced diabetic mice. Exp Mol Med, 2005. 37(6): p. 513-23.
13. Mattsson, G., et al., Endothelial cells in endogenous and transplanted pancreatic islets: differences in the expression of angiogenic peptides and receptors. Pancreatology, 2006. 6(1-2): p. 86-95.
14. Olsson, R., A. Maxhuni, and P.O. Carlsson, Revascularization of transplanted pancreatic islets following culture with stimulators of angiogenesis. Transplantation, 2006. 82(3): p. 340-7.
15. Oh, S.H., et al., Adult bone marrow-derived cells trans-differentiating into insulin producing cells for the treatment of type I diabetes. Lab Invest, 2004. 84(5): p. 607-17.
16. Hess, D., et al., Bone marrow-derived stem cells initiate pancreatic regeneration. Nat Biotechnol, 2003. 21(7): p. 763-70.
17. Lechner, A., et al., No evidence for significant transdifferentiation of bone marrow into pancreatic beta-cells in vivo. Diabetes, 2004. 53(3): p. 616-23.
18. Mathews, V., et al., Recruitment of bone marrow-derived endothelial cells to sites of pancreatic beta-cell injury. Diabetes, 2004. 53(1): p. 91-8.
19. Dawn, B. and R. Bolli, Adult bone marrow-derived cells: Regenerative potential, plasticity, and tissue commitment. Basic Res Cardiol, 2005. 100(6): p. 494-503.
20. Luo, L., N. Yano, and J.Z. Luo, The molecular mechanism of EGF receptor activation in pancreatic beta-cells by thyrotropin-releasing hormone. Am J Physiol Endocrinol Metab, 2006. 290(5): p. E889-99.
21. Luo, L.G., et al., Effect of preproTRH antisense on thyrotropin-releasing hormone synthesis and viability of cultured rat diencephalic neurons. Endocrine, 2001. 15(1): p. 79-85.
22. Luo, J.Z. and L. Luo, American Ginseng Stimulates Insulin Production and Prevents Apoptosis through Regulation of Uncoupling Protein-2 in Cultured beta Cells. Evid Based Complement Alternat Med, 2006. 3(3): p. 365-72.
23. Cabrera, O., et al., The unique cytoarchitecture of human pancreatic islets has implications for islet cell function. Proc Natl Acad Sci U S A, 2006. 103(7): p. 2334-9.
24. Brissova, M., et al., Assessment of human pancreatic islet architecture and composition by laser scanning confocal microscopy. J Histochem Cytochem, 2005. 53(9): p. 1087-97.
25. Welsh, N., et al., Is there a role for locally produced interleukin-1 in the deleterious effects of high glucose or the type 2 diabetes milieu to human pancreatic islets? Diabetes, 2005. 54(11): p. 3238-44.
26. Storling, J., et al., Nitric oxide contributes to cytokine-induced apoptosis in pancreatic beta cells via potentiation of JNK activity and inhibition of Akt. Diabetologia, 2005. 48(10): p. 2039-50.
27. Paraskevas, S., et al., Cell loss in isolated human.islets occurs by apoptosis. Pancreas, 2000. 20(3): p. 270-6.
28. Amrani, A., et al., IL-1alpha, IL-1beta, and IFN-gamma mark beta cells for Fas-dependent destruction by diabetogenic CD4(+) T lymphocytes. J Clin Invest, 2000. 105(4): p. 459-68.
29. Eizirik, D.L. and M.I. Darville, beta-cell apoptosis and defense mechanisms: lessons from type 1 diabetes. Diabetes, 2001. 50 Suppl 1: p. S64-9.
30. Gurol, A.O., et al., Peritransplant and long-term secretion of interleukin-1beta in cyclosporine treated syngeneic rats allografted with islets of langerhans. Transplant Proc, 2005. b37(5): p. 2375-8.
31. Raida, M., et al., Role of bone morphogenetic protein 2 in the crosstalk between endothelial progenitor cells and mesenchymal stem cells. Int J Mol Med, 2006. 18(4): p. 735-9.
32. Ruscetti, F.W., S. Akel, and S.H. Bartelmez, Autocrine transforming growth factor-beta regulation of hematopoiesis: many outcomes that depend on the context. Oncogene, 2005. 24(37): p. 5751-63.
33. Ozturk, M.A., G.S. Guven, and I.C. Haznedaroglu, How hematopoietic stem cells know and act in cardiac microenvironment for stem cell plasticity? Impact of local renin-angiotensin systems. Med Hypotheses, 2004. 63(5): p. 866-74.
34. Zhang, N., et al., Blood-borne stem cells differentiate into vascular and cardiac lineages during normal development. Stem Cells Dev, 2006. 15(1): p. 17-28.
35. Thatava, T., et al., Chromatin-remodeling factors allow differentiation of bone marrow cells into insulin producing cells. Stem Cells, 2006.
36. Taneera, J., et al., Failure of transplanted bone marrow cells to adopt a pancreatic beta-cell fate. Diabetes, 2006. 55(2): p. 290-6.
37. Kang, E.M., et al., Hematopoietic stem cell transplantation prevents diabetes in NOD mice but does not contribute to significant islet cell regeneration once disease is established. Exp Hematol, 2005. 33(6): p. 699-705.
38. Moriscot, C., et al., Human bone marrow mesenchymal stem cells can express insulin and key transcription factors of the endocrine pancreas developmental pathway upon genetic and/or microenvironmental manipulation in vitro. Stem Cells, 2005. 23(4): p. 594-603.
39. Steele, A. and P. Steele, Stem cells for repair of the heart. Curr Opin Pediatr, 2006. 18(5): p. 518-523.
40. Le Blanc, K. and O. Ringden, Mesenchymal stem cells: properties and role in clinical bone marrow transplantation. Curr Opin Immunol, 2006. 18(5): p. 586-91.
41. Fazel, S., et al., Cardioprotective c-kit+ cells are from the bone marrow and regulate the myocardial balance of angiogenic cytokines. J Clin Invest, 2006. 116(7): p. 1865-77.
42. Dell'Agnola, C., et al., Hematopoietic stem cell transplantation does not restore dystrophin expression in Duchenne muscular dystrophy dogs. Blood, 2004. 104(13): p. 4311-8.
43. Chien, K.R., Lost and found: cardiac stem cell therapy revisited. J Clin Invest, 2006. 116(7): p. 1838-40.
44. Wollert, K.C. and H. Drexler, Cell-based therapy for heart failure. Curr Opin Cardiol, 2006. 21(3): p. 234-9.
45. Tang, Y.L., et al., Autologous mesenchymal stem cell transplantation induce VEGF and neovascularization in ischemic myocardium. Regul Pept, 2004. 117(1): p. 3-10.
46. Carvalho, K.A., et al., Proliferation of bone marrow mesenchymal stem cells, skeletal muscle cells and co-culture of both for cell myocardium therapy in Wistar rats. Transplant Proc, 2006. 38(6): p. 1955-6.
47. Bin, F., et al., Construction of tissue-engineered homograft bioprosthetic heart valves in vitro. Asaio J, 2006. 52(3): p. 303-9.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A method for culturing islet β cell comprising culturing an islet β cell with a bone marrow cell *in vitro* for a sustained period of time sufficient to preserve islet β cells such that the insulin releasing function of the cells is preserved, wherein the sustained period is at least about 30 days.

2. A method for pancreatic islet β cell expansion *in vitro* comprising, culturing an islet β cell with a bone marrow cell for a sustained period of time sufficient to preserve β cells such that the insulin releasing function of the cells is preserved, wherein the sustained period is at least about 30 days.

3. A method for increasing islet β cell viability, improved islet β cell function, and/or improved β cell morphology comprising culturing an islet β cell with a bone marrow cell *in vitro* for a sustained period of time sufficient to preserve islet β cells such that the insulin releasing function of the cells is preserved, wherein the sustained period is at least about 30 days.

4. The method of any one of claims 1 to 3, wherein the sustained period is at least about 60 days; at least about 90 days; or at least about 120 days.

5. The method of any one of claims 1 to 4, wherein culturing an islet β cell with a bone marrow cell increases islet β cell viability, improves islet β cell function, and/or improves islet β cell morphology as compared to an islet β cell cultured in the absence of a bone marrow cell.

6. The method of claim 5, wherein cell function is determined by a basal insulin secretion assay or by a glucose induced insulin secretion assay.

7. The method of claim 5, wherein increased islet β cell viability, improved islet β cell function, and/or improved islet β cell morphology are achieved for the sustained period.

8. The method of any one of claims 1-7, wherein the method:
reduces apoptosis of an islet β cell in culture with a bone marrow cell as compared to an islet β cell cultured in the absence of a bone marrow cell;
reduces cytokine release from an islet β cell in culture with a bone marrow cell as compared to an islet β cell cultured in the absence of a bone marrow cell; or
increases the expression of at least one endocrine cell specific gene.

9. The method of claim 8, wherein the cytokine is IL-1 β.

10. The method of claim 8, wherein the endocrine cell specific gene is selected from the group consisting of GCG (glucagon, α-cell gene), INS (insulin, β-cell gene), and SST (somatostatin, γ-cell gene); transcription factors for β cell pancreatic and duodenal homeobox1 (PDX1 or IPF1), Neurogenin 3 (NGN3), paired box gene 6 (PAX6), islet-1 (ISL1), v-maf musculoaponeurotic fibrosarcoma oncogene homolog A (MAFa), and Mist 1.

## Patentansprüche

1. Verfahren zum Kultivieren von β-Inselzellen, wobei man eine β-Inselzelle mit einer Knochenmarkszelle *in vitro* über einen längeren, für die Erhaltung der β-Inselzellen ausreichenden Zeitraum kultiviert, so dass die Insulin freisetzende Funktion der Zellen erhalten bleibt, wobei der längere Zeitraum wenigstens etwa 30 Tage beträgt.

2. Verfahren zur *in vitro* Expansion von β-Inselzellen der Bauchspeicheldrüse, wobei man eine β-Inselzelle mit einer Knochenmarkszelle über einen längeren, für die Erhaltung der β-Inselzellen ausreichenden Zeitraum kultiviert, so dass die Insulin freisetzende Funktion der Zellen erhalten bleibt, wobei der längere Zeitraum wenigstens etwa 30 Tage beträgt.

3. Verfahren zur Erhöhung der Lebensfähigkeit von β-Inselzellen, zur Verbesserung der β-Inselzellfunktion und/oder zur Verbesserung der β-Zellmorphologie, wobei man eine β-Inselzelle mit einer Knochenmarkszelle *in vitro* über einen längeren, für die Erhaltung der β-Inselzellen ausreichenden Zeitraum kultiviert, so dass die Insulin freisetzende Funktion der Zellen erhalten bleibt, wobei der längere Zeitraum wenigstens etwa 30 Tage beträgt.

4. Verfahren nach einem der Ansprüche 1 bis 3, wobei der längere Zeitraum wenigstens etwa 60 Tage; wenigstens etwa 90 Tage; oder wenigstens etwa 120 Tage beträgt.

5. Verfahren nach einem der Ansprüche 1 bis 4, wobei die Kultivierung einer β-Inselzelle mit einer Knochenmarkszelle die Lebensfähigkeit der β-Inselzelle erhöht, die β-Inselzellfunktion verbessert und/oder die β-Inselzellmorphologie verbessert, im Vergleich zu einer β-Inselzelle, die in Abwesenheit einer Knochenmarkszelle kultiviert wurde.

6. Verfahren nach Anspruch 5, wobei die Zellfunktion anhand eines Tests zur basalen Insulinsekretion oder eines Tests zur glukoseinduzierten Insulinsekretion bestimmt wird.

7. Verfahren nach Anspruch 5, wobei die erhöhte Lebensfähigkeit der β-Inselzellen-, verbesserte β-Inselzellfunktion und/oder verbesserte β-Inselzellmorphologie für einen längeren Zeitraum erhalten werden.

8. Verfahren nach einem der Ansprüche 1-7, wobei das Verfahren:
die Apoptose einer β-Inselzelle in Kultur mit einer Knochenmarkszelle im Vergleich zu einer in Abwesenheit einer Knochenmarkszelle kultivierten β-Inselzelle verringert; die Zytokinfreisetzung einer β-Inselzelle in Kultur mit einer Knochenmarkszelle im Vergleich zu einer in Abwesenheit einer Knochenmarkszelle kultivierten β-Inselzelle verringert; oder die Expression wenigstens eines Gens, dass für eine endokrine Zelle spezifisch ist, erhöht.

9. Verfahren nach Anspruch 8, wobei das Zytokin IL-1-β ist.

10. Verfahren nach Anspruch 8, wobei das Gen, das für eine endokrine Zelle spezifisch ist, ausgewählt ist unter GCG (Glucagon, a-Zellgen), INS (Insulin, β-Zellgen), und SST (Somatostatin, y-Zellgen); Transskriptionsfaktoren für pankreatische und duodenale Homeobox1-β-Zellen (PDX1 oder IPF1), Neurogenin 3 (NGN3), Paarboxgen 6 (PAX6), Insel-1 (ISL1), muskoloaponeurotisches v-maf Fibrosarkom-Onkogenhomolog A (MAFa), und Mist 1.

## Revendications

1. Procédé de culture de cellule β d'îlot comprenant la culture d'une cellule β d'îlot avec une cellule de moelle osseuse *in vitro* pendant une durée prolongée suffisante pour préserver les cellules β d'îlot de façon à ce que la fonction de libération d'insuline des cellules soit préservée, dans lequel la durée prolongée est d'au moins environ 30 jours.

2. Procédé pour l'expansion de cellules β d'îlot pancréatique *in vitro* comprenant la culture d'une cellule β d'îlot avec une cellule de moelle osseuse pendant une durée prolongée suffisante pour préserver les cellules β de façon à ce que la fonction de libération d'insuline des cellules soit préservée, dans lequel la durée prolongée est d'au moins environ 30 jours.

3. Procédé pour l'augmentation de la viabilité de cellules β d'îlot, une fonction améliorée de cellules β d'îlot et/ou une morphologie améliorée de cellules β comprenant la culture d'une cellule β d'îlot avec une cellule de moelle osseuse *in vitro* pendant une durée prolongée suffisante pour préserver les cellules β d'îlot de façon à ce que la fonction de libération d'insuline des cellules soit préservée, dans lequel la durée prolongée est d'au moins environ 30 jours.

4. Procédé selon l'une quelconque des revendications 1 à 3, dans lequel la durée prolongée est d'au moins environ 60 jours ; d'au moins environ 90 jours ; ou d'au moins environ 120 jours.

5. Procédé selon l'une quelconque des revendications 1 à 4, dans lequel la culture d'une cellule β d'îlot avec une cellule de moelle osseuse augmente la viabilité de la cellule β d'îlot, améliore la fonction de la cellule d'îlot et/ou améliore la morphologie de la cellule β d'îlot par comparaison avec une cellule β d'îlot cultivée en l'absence d'une cellule de moelle osseuse.

6. Procédé selon la revendication 5, dans laquelle la fonction cellulaire est déterminée par un essai de sécrétion basale d'insuline ou par un essai de sécrétion d'insuline induite par le glucose.

7. Procédé selon la revendication 5, dans lequel une viabilité accrue de la cellule β d'îlot, une fonction améliorée de la cellule β d'îlot et/ou une morphologie améliorée de la cellule β d'îlot sont réalisées pendant la durée prolongée.

8. Procédé selon l'une quelconque des revendications 1 à 7, le procédé : réduisant l'apoptose d'une cellule β d'îlot en culture avec une cellule de moelle osseuse par comparaison avec une cellule β d'îlot cultivée en l'absence d'une cellule de moelle osseuse ; réduisant la libération de cytokine d'une cellule β d'îlot en culture avec une cellule de moelle osseuse par comparaison avec une cellule β d'îlot cultivée en l'absence d'une cellule de moelle osseuse ; ou augmentant l'expression d'au moins un gène spécifique de cellules endocrines.

9. Procédé selon la revendication 8, dans lequel la cytokine est IL-1 β.

10. Procédé selon la revendication 8, dans lequel le gène spécifique de cellules endocrines est choisi dans le groupe constitué par GCG (glucagon, gène des cellules α), INS (insuline, gène des cellules β) et SST (somatostatine, gène des cellules γ) ; les facteurs de transcription pour l'homéoboîte 1 pancréatique et duodénale des cellules β (PDX1 ou IPF1), la neurogénine 3 (NGN3), le gène à boîte paired 6 (PAX6), l'îlot 1 (ISL1), l'homologue oncogène du fibrosarcome musculo-aponévrotique v-maf A (MAFa) et Mist 1.
